# EUROPEAN PATENT SPECIFICATION

(11) **EP 1 269 860 B1**
(45) Date of publication and mention of the grant of the patent: **26.10.2005**
(21) Application number: 02078716.4
(22) Date of filing: 04.06.1999
(51) Int. Cl.: A23L 1/308, A61K 45/06, A61K 38/54

(54) **Pharmaceutical composition**
Pharmazeutische Zusammensetzung
Composition pharmaceutique

(30) Priority: 16.06.1998 EP 98830365
(43) Date of publication of application: 02.01.2003
(62) Divisional of application: 99201794.7
(73) Proprietor: Zohoungbogbo, Mathias Christian, 10040 Rivalta di Torino - Torino (IT); Gobbato, Rosa Anna, 10040 Rivalta di Torino - Torino (IT)
(72) Inventor: Zohoungbogbo, Mathias Christian, 10040 Rivalta di Torino - Torino (IT)
(74) Representative: Long, Giorgio

(56) References cited:
- FR-A- 2 710 245
- GB-A- 1 508 940
- US-A- 5 106 634

## Description

This invention relates to a pharmaceutical composition for preventing and treating the side-effects which can occur during a ketogenic diet.

It is well known that in the developed nations and even in the developing nations the percentage of individuals having excess weight if not obesity problems is constantly and progressively increasing. This has important consequences both for the health of individuals and for the overall health cost of the various nations, because it has been amply demonstrated that obesity or even being overweight are important associated causes of cardiovascular and metabolic diseases, such as myocardial infarction, stroke, type II diabetes, etc.

The dietetic measures generally proposed by dieticians to combat and/or prevent excess weight mainly consist of low-calorie or low-fat regimes.

Another widely recommended measure for the control of body weight which is even advocated by the mass media (periodicals, television, etc.) is adoption of the famous "Mediterranean diet", based on foods rich in complex carbohydrates such as pasta, rice, bread and the like.

It is, however, a fact that the widespread use of low-calorie diets and the Mediterranean diet has not in fact resulted in any statistically significant change in the percentage of obese or overweight persons; there is instead a progressive increase in this percentage.

A low-calorie diet can in fact have some temporary effect on reducing body weight but this cannot be maintained for a long period, either because it results in general weakening of the body, or because over the long term it is rejected by the individual because of the monotony of the flavours of the fopd making it up (essentially meat, fish and greens).

The so-called "Mediterranean diet" is in fact only suitable for maintaining the right weight and the right form in individuals who are engaged in vigorous physical activity. Persons who are engaged in essentially sedentary work can on the other hand experience an increase in weight and an accumulation of lipids when they feed themselves on foods based essentially on carbohydrates.

To overcome the above mentioned drawbacks deriving from low-calorie and "Mediterranean" diets, it has been proposed to totally or partially eliminate carbohydrates from overweight persons' diet. Said kind of diet is usually known in the art as ketogenic diet. The ketogenic diet satisfies always one's appetite and brings always to a loss in weight, but may also cause several side-effects such as, for instance, hyperuricemia, hyperglycemia, hypercholesterolemia, hypertriglyceridemia, hepatic-pancreatic alterations, mental disorders, etc.

Therefore, the problem underlying this invention is that of providing a composition for preventing and treating the above mentioned side-effects which can occur in a ketogenic diet.

FR 2 710 245 discloses a composition comprising benfluorex, papaine, ursodesoxycholic acid, chlorazepate, betaine, carnitine, hiacine and lecithine.

Said problem is solved by a pharmaceutical composition as recited in the appended claims.

In particular, said pharmaceutical composition A comprises an hypocholesterolemic agent, a hypotriglyceridemic agent, a lipasic and proteasic agent, a hypoglycemic agent and a hydrocoleretic agent.

The hypocholesterolemic agent is preferably selected from the group consisting of 2-[alpha-methyl-(trifluaromethyl)phenethylamine)-ethanolbenzoate (benfluorex) and 3-alpha-7B-dihydroxy-5B-colan-24 oic acid (ursodesoxycolic acid).

The hypotriglyceridic agent is preferably 2-[alphamethyl-(trifluoromethyl)phenethylamine]-ethanolbenzoate (benfluorex) which is preferably present in a global amount from 7% to 23% in weight with respect of the total amount of the composition A.

The lipase and protease agent is preferably total lyophilized pancreas (Pancreatine IX F.U.) which is preferably present in an amount from 27% to 43% in weight with respect of the total amount of the composition A.

The hypoglycemic agent is preferably selected from the group consisting of biguanides, said biguanides being preferably 1,1-dimethylbiguanide (metformine) which is preferably present in an amount from 36% to 41% in weight with respect to the total amount of the composition A.

The hydrocoleretic agent is preferably selected from the group consisting of 3,7,12-triose-5B-colan-24 oic acid (Na dehydrocolate) and 3alfa-7B-dihydroxy-5B-colan-24 oic acid (ursodesoxycolic acid). When Na dehydrocolate is present, it is preferably in an amount from 9% to 14% in weight with respect of the total amount of the composition A. When ursodesoxycolic acid is present, it is preferably in an amount from 14% to 17% in weight with respect to the total amount of the composition A.

The composition may additionally comprise an hypouricemic agent. Said agent is preferably centella asiatica purified triterpenes which may be preferably present in a ratio from 0,04:1 to 0,5:1 in weight with respect to the total amount of the composition A.
Moreover, the composition may comprise a radical scavenger agent. Preferably said agent is selenium which may be preferably present in a ratio from 0,0001:1 to 0,0003:1 in weight with respect to the total amount of the composition A.

The composition may also comprise a sympatholytic agent. Said agent is preferably methyl 17-alfa-hydroxy-yohimbane-16-alfa-carboxylate (yohimbine) which may be preferably present in a ratio from 0,0009:1 to 0,007:1 in weight with respect of the total amount of the composition A.

The composition may additionally comprise a sympathicomimetic agent. Said agent is selected from the group consisting of phendimetrazine bitartrate and phendimetrazine pamoate, which may be preferably present in a ratio from 0,004:1 to 0,1:1 in weight with respect to the total amount of the composition A..

Furthermore, the composition may comprise at least one vitamin, preferably said at least one vitamin is selected in the group consisting of vitamin A, vitamin B₁, vitamin B₆, vitamin E and vitamin C.

When vitamin A is present, it is in a ratio from 0,4:1 to 1,8:1 in weight with respect to the total amount of the composition A.

When vitamin B₁ is present, it is in a ratio from 0,002:1 to 0,007:1 in weight with respect to the total amount of the composition A.

When vitamin B₆ is present, it is in a ratio from 0,04:1 to 0,2:1 in weight with respect to the total amount of the composition A.

When vitamin E is present, it is in a ratio from 0,09:1 to 1:1 in weight with respect to the total amount of the composition A.

When vitamin C is present, it is in a ratio from 0,09:1 to 0,3:1 in weight with respect to the total amount of the composition A.

The composition A may further comprise at least one diet adjuvant selected from the group consisting of a sedative-ansiolytic agent, an anorectic agent and a lipolytic agent.

The sedative-ansiolytic agent is preferably a benzodiazepine, most preferably 7-chloro-2,3-dihydro-2-oxo-5-phenyl-1H-1,4-benzodiazepine (dipotassium chlorazepate) which is preferably present in a ratio from 0,0004:1 to 0,03:1 in weight with respect to the total amount of the composition A.

The anorectic agent is preferably selected from the group consisting of 1-phenyl-2-diethylamine-1-proparone hydrochloride (diethylpropione chlorohydrate), fenfluramine chlorohydrate, D-fenfluramine chlorohydrate. Said anorectic agent is preferably present in an amount from 0,002:1 to 0,1:1 in weight with respect to the total amount of the composition A.

The lipolytic agent is preferably selected from the group consisting of analogues of tiroxine, most preferably said agent is 3,5,3'triiodiotiroacetic acid which is preferably present in a ratio from 0,0002:1 to 0,003:1 in weight with respect to the total amount of the composition A.

All the above agents which can be comprised into the composition A, are usually commercially available.

In particular, diethylpropione chlorohydrate is an anorectic agent used as adjuvant in the treatment of obesity. It shows the same activity of the amphetamine, but with less effects upon the CNS (Central Nervous System) and upon cardiovascular system. Therefore, it is preferable for hypertensive, cardiopath, diabetic and elderly persons.

Fenfluramine and D-fenfluramine are anorectic agents used as adjuvants in the treatment of obesity, which show a psycho-stimulating effect.

Benfluorex is an hypocholesterolemic and hypotriglyceridemic agent which also shows anorectic and hypoglycemic activities.

Centella asiatic is a light diuretic, antirheumatic and peripheral vasodilative agent used in the treatment of the pre-varicose status.

Pancreatine IX F.U. is total lyophilized pancreas used in the treatment of pancreatic insufficiency. Metformine is an hypoglycemic agent used in the treatment of diabetes specially if in the presence of obesity. It has been also demonstrated a positive effect of metformine on lipid metabolism and on decrease in weight as well as an anorectic activity on diabetic obese patients.

Na dehydrocolate is an hydrocoleretic agent which causes the low-density biliary secretion. Ursodesoxycolic acid is an agent used to fluidify the bile. It has an anti-cholesterol activity, too.
Selenium is an element used as radical scavenger. It is also used to prevent Kershan disease and as adjuvant during chemotherapy.

Yohimbine is a sympatholytic agent as well as a mydriatic agent. It has been used in the treatment of impotence.

Vitamin A is used to cure hypovitaminosis, malabsorption, malnutrition and to treat dermatological diseases.

Vitamin B₁ is used in the treatment and prophylaxis of thiamine deficit, in the treatment of gravidic hyperemesis, neuritis caused by pellagra and during pregnancy.

Vitamin B₆ is used in the treatment and prophylaxis of pyridoxine deficit, in the treatment of anaemia, convulsions, gravidic and radiant hyperemesis in the prophylaxis of peripheral neuritis.

Vitamin E is used to cure hypovitaminosis, lipid malabsorption and is used as anti-oxidant for premature infants.

Vitamin C is used to treat ascorbic acid deficit. Triiodiotiroacetic acid is a thyroid hormone which shows a lipolytic activity.

Dipotassium chlorazepate is a sedative-ansiolytic agent which has the pharmacological activity of the benzodiazepine.

Fendimetrazine bitartrate and pamoate are sympathomimetic agent. They also shows a central anorectic activity.

The composition of the drug and the doses will vary with the conditions of the patients to be treated. Therefore, specific dosages regiments should be adjusted to the individual need and the professional judgement of the person administering or supervising the administration of the aforesaid composition. The dosages may be administered at once or may be divided into a number of smaller doses to be administered at varying intervals of time.

Said agents have been selected to be combined in a synergetic way such that to improve their single pharmaceutical properties and, at the same time, not interfering one with the others.

The advantage provided by said pharmaceutical composition is that of preventing or treating the above mentioned side-effects which can occur in a diet lacking in carbohydrates.

Moreover, by means of a combination of a ketogenic diet and of the pharmaceutical composition according to the invention, it is always possible to loose weight without the person having to suffer deprivation of foodstuffs such as pasta, bread and bakery product.

The pharmaceutical composition of the invention may additionally contain excipient and optionally other auxiliary agents, if necessary.

The composition of the present invention can be administered in different pharmaceutical formulation, the precise nature of which will depend upon the chosen route of administration.

Thus, solid compositions for orally administration include compressed tablets, dispersible powders, granules and capsules.

In tablets, the active components are admixed with at least one inert diluent such as lactose, starch, mannitol, microcrystalline cellulose or calcium phosphate; granulating and disintegrating agents for examples corn starch, gelatine, microcrystalline cellulose or polyvinylpyrrolidone; lubricating agents such as magnesium stearate, stearic acid or talc. The tablets may be coated by known techniques to delay disintegration and absorption in the gastrointestinal tract and, thereby, provide a sustained action over a long period. Gastric film-coated or enteric film-coated can be made with sugar, gelatin, hydroxypropylcellulose or acrylic resins.

Formulation for oral use may also be presented as hard capsules of absorbable material, such as gelatin, wherein the active ingredient is mixed with an inert diluent and lubricating agents, or pasty materials, such as ethoxylated saturated glycerides.

Soft gelatine capsules are possible wherein the active ingredient is mixed with water or an oil medium, for example, peanut oil, liquid paraffin or olive oil. Dispersible powders and granules suitable for preparation of a suspension by the addition of water provide the active ingredient in admixture with dispersing or wetting agent, a suspending agent, such as sodium carboxymethylcellulose, sodium alginate, polyvinylpirrolidone, gum tragacanth, xantham gum, gum acacia and one or more preservatives, such as methyl or n-propyl-p-hydroxybenzoate. Additional excipients, for example sweetening, flavouring and colouring agents may also be present.

Liquid composition for oral administration include emulsions, solutions, suspensions, syrups and elixirs containing commonly used inert diluents, such as distilled water, ethanol, sorbitol, glycerol or propylene glycol. Such compositions may comprise adjuvants such as wetting agents, suspending agents, sweetening, flavouring, perfuming, preserving agents and buffers.

The pharmaceutical composition of the present invention may also be administered in the form of suppositories for rectal administration of the drug.

The dosage and frequency of dose may vary depending upon symptoms, age and body weight of the patient, as well as upon the route of administration, but in general the composition of the present invention may be administered orally in doses of from 7g to 23g a day to a patient of the average weight of 70kg.

The above composition may be prepared in the form of a kit. Said kit may comprise the composition as such or its components separately prepared, for instance, in the form of different capsules. In this manner it is possible to combine different compounds and/or their different amount depending on the patient to be treated.

It is therefore a further object of the present invention a kit of parts for sequential, simultaneous or separate administration as recited in the appended claims.

The pharmaceutical composition according to this invention will be further described with reference to the examples provided below merely by way of non-restrictive illustration.

The following compositions are preferably prepared in the form of a gelatine capsule.

### EXAMPLE 1

Preparation of a gelatine capsule. The following components are admixed, optionally with suitable excipients to reach the capacity of the capsule: the capsule is then filled and sealed.

| | | |
|---|---|---|
| Diethylpropione chlorohydrate | mg. | 20 |
| Fenfluramine | mg. | 4 |
| Benfluorex | mg. | 50 |
| Triiodiotiracetic acid | mg. | 0,4 |
| Pancreatine IX F.U. | mg. | 120 |
| Metformine | mg. | 200 |
| Na dehydrocolate | mg. | 40 |

### EXAMPLE 2

Preparation of two capsules A and B which are part of a kit and which comprise different components to take in combination.

| Capsule A | | |
|---|---|---|
| Fenfluoramine | mg. | 4 |
| Benfluorex | mg. | 50 |

| Capsule B | | |
|---|---|---|
| Triiodiotiracetic acid | mg. | 0,8 |
| Pancreatine IX F.U. | mg. | 240 |
| Metformine | mg. | 370 |
| Na dehydrocolate | mg. | 80 |
| Dipotassium chlorazepate | mg. | 2 |

### EXAMPLE 3

Preparation of a capsule A and a capsule B which are part of a kit and which comprise different compounds to take in combination.

| Capsule A | | |
|---|---|---|
| Diethylpropione chlorohydrate | mg. | 35 |
| Benfluorex | mg. | 150 |
| Vit. C | mg. | 100 |
| Vit. E | mg. | 100 |
| Vit B₆ | mg. | 50 |

| Capsule B | | |
|---|---|---|
| Pancreatine IX F.U. | mg. | 240 |
| Metformine | mg. | 370 |
| Na dehydrocolate | mg. | 80 |
| Dipotassium chlorazepate | mg. | 4 |

## Claims

1. Pharmaceutical composition (A) comprising:
an hypocholesterolemic agent, said hypocholesterolemic agent being preferably selected from the group consisting of benfluorex and ursodesoxycolic acid;
an hypotriglyceride agent, said hypotriglyceride agent being preferably benfluorex;
a lipasic and proteasic agent, said lipasic and proteasic agent being preferably pancreatine IX F.U.;
an hypoglycemic agent, said hypoglycemic agent being preferably metformine and
an hydrocoleretic agent, said hydrocoleretic agent being preferably selected from the group consisting of Na dehydrocloate and ursodesoxycolic acid.

2. Pharmaceutical composition according to claim 1, wherein:
said benfluorex is present in a global amount from 7% to 23% in weight of the total amount of the composition (A);
said pancreatine IX F.U. is present in an amount from 27% to 43% in weight of the total amount of the composition (A);
said metformine is present in an amount from 36% to 41% in weight of the total amount of the composition (A); said Na dehydrocloate is present in an amount from 9% to 14% of the total amount of the composition (A).

3. Pharmaceutical composition according to claim 1, wherein:
said benfluorex is present in a global amount from 7% to 18% in weight of the total amount of the composition (A);
said pancreatine IX F.U. is present in an amount from 22% to 43% in weight of the total amount of the composition (A);
said metformine is present in an amount from 33% to 36% in weight of the total amount of the composition (A); said ursodesoxycolic acid is present in an amount from 14% to 17% of the total amount of the composition (A).

4. Pharmaceutical composition according to any of the claims 1 to 3, wherein the composition (A) further comprises:
- an hypouricemic agent, said hypouricemia agent being preferably centella asiatica purified triterpenes; and/or
- a radical scavenger agent, said radical scavenger agent being preferably selenium; and/or
- a sympatholytic agent, said sympatholytic agent being preferably yohinbine; and/or
- a sympathicomimetic agent, said sympathicomimetic agent being selected from the group consisting of phendimetrazinum bitartrate and phendimetrazinum pamoate; and/or
- at least one vitamin, said at least one vitamin being selected from the group consisting of vitamin A, vitamin B₁, vitamin B₆, vitamin E and vitamin C.

5. Pharmaceutical composition according to any of the claims 1 to 4, wherein the composition further comprises at least one diet adjuvant selected from the group consisting of sedative-ansiolytic agents, anorectic agents and lipolytic agents.

6. Pharmaceutical composition according to claim 4 or 5, comprising:
- centella asiatica purified triterpenes in a ratio from 0,04:1 to 0,5:1 in weight with respect to the total weight of composition (A) of claims 1 to 3; and/or
- selenium in a ration from 0,09:1 to 0,3:1 in weight with respect to the total weight of composition (A) of claims 1 to 3; and/or
- yohimbine in a ratio from 0,0009:1 to 0,007:1 in weight with respect to the total weight of composition (A) of claims 1 to 3; and/or
- phendimetrazine bitartarate or phendimetrazine pamoate in a ratio from 0,004:1 to 0,13:1 in weight with respect to the total weight of composition (A) of claims 1 to 3; and/or
- vitamin A in a ratio from 0,5:1 to 1,8:1 in weight with respect to the total weight of composition (A) of claims 1 to 3; and/or
- vitamin B₁ in a ratio from 0,002:1 to 0,007:1 in weight with respect to total weight of composition (A) of claims 1 to 3; and/or
- vitamin B₆ in a ratio from 0,05:1 to 0,2:1 in weight with respect to the total weight of composition (A) of claims 1 to 3; and/or
- vitamin E in a ratio from 0,09:1 to 1:1 in weight with respect to the total weight of composition (A) of claims 1 to 3; and/or
- vitamin C in a ratio from 0,09:1 to 0,3:1 in weight with respect to the total weight of composition (A) of claims 1 to 3.

7. Pharmaceutical composition according to claim 5 or 6, wherein:
- said sedative-ansiolityc agent is preferably a benzodiazepine, most preferably dipotassium chlorazepate in a ratio from 0,0005:1 to 0,03:1 in weight with respect to the total weight of composition (A) of claims 1 to 3; and/or
- said anorectic agent is preferably selected from the group consisting of diethylpropione chlorhydrate, fenfluramine chlohydrate, D- fenfluramine chlohydrate, said anorectic agent being present in a ratio from 0,002:1 to 1,3:1 in weight with respect to the total weight of composition (A) of claims 1 to 3; and/or
- said lipilityc agent is preferably selected from the group consisting of analogues of tiroxine, most preferably being triiodiotiroacetic acid which is present in a ratio from 0,0002:1 to 0,003:1 in weight with respect to the total weight of composition (A) of claims 1 to 3.

8. Pharmaceutical composition according to any of claims 1 to 7, which further comprises suitable pharmaceutically acceptable excipients.

9. Pharmaceutical composition according to any of claims 1 to 8, wherein said composition is a solid composition for oral administration comprising compressed tablets, dispersible powders, granules and gelatine capsules.

10. Kit of parts for the simultaneous, sequential or separated administration, comprising:
an hypocholesterolemic agent, said hypocholesterolemic agent being preferably selected from the group consisting of benfluorex and ursodesoxycolic;
an hypotriglyceride agent, said hypotriglyceride agent being preferably benfluorex;
a lipasic and proteasic agent, said lipasic and proteasic agent being preferably pancreatine IX F.U.;
an hypoglycemic agent, said hypoglycemic agent being preferably metformine and
an hydrocoleretic agent, said hydrocoleretic agent being preferably selected from the group consisting of Na dehydrocloate and ursodesoxycolic acid.

11. Kit according to claim 10, which further comprises:
- an hypouricemic agent, said hypouricemic agent being preferably centella asiatica purified triterpenes; and/or
- a radical scavenger agent, said radical scavenger agent being preferably selenium; and/or
- a sympatholytic agent, said sympatholytic agent being preferably yohinbine; and/or
- a sympathicomimetic agent, said sympathicomimetic agent being selected from the group consisting of phendimetrazinum bitartarate and phendimetruzinum pamoate; and/or
- at least one vitamin, said at least one vitamin being selected from the group consisting of vitamin A, vitamin B₁, vitamin B₆, vitamin E and vitamin C; and/or
- at least one adjuvant selected from the group consisting of a sedative-ansiolytic agent, an anorectic agent and a lipolytic agent.

12. Kit according to claim 11, wherein:
- said sedative-ansiolityc agent is preferably a benzodiazepine, most preferably dipotassium chlorazepate;
- said anorectic agent is preferably selected from the group consisting of diethylpropione chlorhydrate, fenfluramine chlohydrate, D- fenfluramine chlohydrate;
- said lipolytic agent is preferably selected from the group consisting of analogues of tiroxine, most preferably being triiodiotiroacetic acid.

13. Use of a composition according to any one of the claims 1 to 9 for the manufacture of a medicament for treating side-effects of ketogenic diets.

## Patentansprüche

1. Pharmazeutische Zusammensetzung (A), umfassend:
ein hypocholesterinämisches Mittel, wobei das hypocholesterinämische Mittel vorzugsweise ausgewählt ist aus der Gruppe bestehend aus Benfluorex und Ursodesoxycholsäure;
ein hypotriglyceridämisches Mittel, wobei das hypotriglyceridämische Mittel vorzugsweise Benfluorex ist;
ein Lipase- und Proteasemittel, wobei das Lipase- und Proteasemittel vorzugsweise Pankreatin IX F.U. ist;
ein hypoglykämisches Mittel, wobei das hypoglykämische Mittel vorzugsweise Metformin ist, und
ein hydrocholeretisches Mittel, wobei das hydrocholeretische Mittel vorzugsweise ausgewählt ist aus der Gruppe bestehend aus Na-Dehydrocholat und Ursodesoxycholsäure.

2. Pharmazeutische Zusammensetzung nach Anspruch 1, worin:
das Benfluorex in einer Gesamtmenge von 7 bis 23 Gew.-%, bezogen auf die Gesamtmenge der Zusammensetzung (A), vorhanden ist;
das Pankreatin IX F.U. in einer Menge von 27 bis 43 Gew.-%, bezogen auf die Gesamtmenge der Zusammensetzung (A), vorhanden ist;
das Metformin in einer Menge von 36 bis 41 Gew.-%, bezogen auf die Gesamtmenge der Zusammensetzung (A), vorhanden ist;
das Na-Dehydrocholat in einer Menge von 9 bis 14 Gew.-%, bezogen auf die Gesamtmenge der Zusammensetzung (A), vorhanden ist.

3. Pharmazeutische Zusammensetzung nach Anspruch 1, worin:
das Benfluorex in einer Gesamtmenge von 7 bis 18 Gew.-%, bezogen auf die Gesamtmenge der Zusammensetzung (A), vorhanden ist;
das Pankreatin IX F.U. in einer Menge von 22 bis 43 Gew.-%, bezogen auf die Gesamtmenge der Zusammensetzung (A), vorhanden ist;
das Metformin in einer Menge von 33 bis 36 Gew.-%, bezogen auf die Gesamtmenge der Zusammensetzung (A), vorhanden ist;
die Ursodesoxycholsäure in einer Menge von 14 bis 17 Gew.-%, bezogen auf die Gesamtmenge der Zusammensetzung (A), vorhanden ist.

4. Pharmazeutische Zusammensetzung nach einem der Ansprüche 1 bis 3, worin die Zusammensetzung (A) ferner umfasst:
- ein hypourikämisches Mittel, wobei das hypourikämische Mittel vorzugsweise gereinigte Triterpene aus Centella asiatica sind; und/oder
- einen Radikalfänger, wobei der Radikalfänger vorzugsweise Selen ist; und/oder
- ein sympatholytisches Mittel, wobei das sympatholytische Mittel vorzugsweise Yohimbin ist; und/oder
- ein sympathikomimetisches Mittel, wobei das sympathikomimetische Mittel ausgewählt ist aus der Gruppe bestehend aus Phendimetrazin-bitartrat und Phendimetrazin-pamoat; und/oder
- wenigstens ein Vitamin, wobei das wenigstens eine Vitamin ausgewählt ist aus der Gruppe bestehend aus Vitamin A, Vitamin B₁, Vitamin B₆, Vitamin E und Vitamin C.

5. Pharmazeutische Zusammensetzung nach einem der Ansprüche 1 bis 4, wobei die Zusammensetzung ferner wenigstens ein Diätadjuvans, ausgewählt aus der Gruppe bestehend aus sedativen und angstlösenden Mitteln, Appetitzüglern und lipolytischen Mitteln, umfasst.

6. Pharmazeutische Zusammensetzung nach Anspruch 4 oder 5, umfassend:
- gereinigte Triterpene aus Centella asiatica in einem Gewichtsverhältnis von 0,04:1 bis 0,5:1, bezogen auf das Gesamtgewicht der Zusammensetzung (A) der Ansprüche 1 bis 3; und/oder
- Selen in einem Gewichtsverhältnis von 0,09:1 bis 0,3:1, bezogen auf das Gesamtgewicht der Zusammensetzung (A) der Ansprüche 1 bis 3; und/oder
- Yohimbin in einem Gewichtsverhältnis von 0,0009:1 bis 0,007:1, bezogen auf das Gesamtgewicht der Zusammensetzung (A) der Ansprüche 1 bis 3; und/oder
- Phendimetrazin-bitartrat oder Phendimetrazin-pamoat in einem Gewichtsverhältnis von 0,004:1 bis 0,13:1, bezogen auf das Gesamtgewicht der Zusammensetzung (A) der Ansprüche 1 bis 3; und/oder
- Vitamin A in einem Gewichtsverhältnis von 0,5:1 bis 1,8:1, bezogen auf das Gesamtgewicht der Zusammensetzung (A) der Ansprüche 1 bis 3; und/oder
- Vitamin B₁ in einem Gewichtsverhältnis von 0,002:1 bis 0,007:1, bezogen auf das Gesamtgewicht der Zusammensetzung (A) der Ansprüche 1 bis 3; und/oder
- Vitamin B₆ in einem Gewichtsverhältnis von 0,05:1 bis 0,2:1, bezogen auf das Gesamtgewicht der Zusammensetzung (A) der Ansprüche 1 bis 3; und/oder
- Vitamin E in einem Gewichtsverhältnis von 0,09:1 bis 1:1, bezogen auf das Gesamtgewicht der Zusammensetzung (A) der Ansprüche 1 bis 3; und/oder
- Vitamin C in einem Gewichtsverhältnis von 0,09:1 bis 0,3:1, bezogen auf das Gesamtgewicht der Zusammensetzung (A) der Ansprüche 1 bis 3.

7. Pharmazeutische Zusammensetzung nach Anspruch 5 oder 6, worin:
- das sedative und angstlösende Mittel vorzugsweise ein Benzodiazepin, am meisten bevorzugt Dikaliumchlorazepat in einem Gewichtsverhältnis von 0,0005:1 bis 0,03:1, bezogen auf das Gesamtgewicht der Zusammensetzung (A) der Ansprüche 1 bis 3 ist; und/oder
- der Appetitzügler vorzugsweise ausgewählt ist aus der Gruppe bestehend aus Diethylpropion-hydrochlorid, Fenfluramin-hydrochlorid, D-Fenfluramin-hydrochlorid, wobei der Appetitzügler in einem Gewichtsverhältnis von 0,002:1 bis 1,3:1, bezogen auf das Gesamtgewicht der Zusammensetzung (A) der Ansprüche 1 bis 3 vorhanden ist; und/oder
- das lipolytische Mittel vorzugsweise ausgewählt ist aus der Gruppe bestehend aus Analoga von Thyroxin, wobei Trüodthyroessigsäure am meisten bevorzugt ist, welche in einem Gewichtsverhältnis von 0,0002:1 bis 0,003:1, bezogen auf das Gesamtgewicht der Zusammensetzung (A) der Ansprüche 1 bis 3, vorhanden ist.

8. Pharmazeutische Zusammensetzung nach einem der Ansprüche 1 bis 7, welche ferner geeignete pharmazeutisch annehmbare Exzipientien umfasst.

9. Pharmazeutische Zusammensetzung nach einem der Ansprüche 1 bis 8, wobei die Zusammensetzung eine feste Zusammensetzung zur oralen Verabreichung ist, umfassend komprimierte Tabletten, dispergierbare Pulver, Körnchen und Gelatinekapseln.

10. Teilekit zur gleichzeitigen, aufeinanderfolgenden oder getrennten Verabreichung, umfassend:
ein hypocholesterinämisches Mittel, wobei das hypocholesterinämische Mittel vorzugsweise ausgewählt ist aus der Gruppe bestehend aus Benfluorex und Ursodesoxycholsäure;
ein hypotriglyceridämisches Mittel, wobei das hypotriglyceridämische Mittel vorzugsweise Benfluorex ist;
ein Lipase- und Proteasemittel, wobei das Lipase- und Proteasemittel vorzugsweise Pankreatin IX F.U. ist;
ein hypoglykämisches Mittel, wobei das hypoglykämische Mittel vorzugsweise Metformin ist, und
ein hydrocholeretisches Mittel, wobei das hydrocholeretische Mittel vorzugsweise ausgewählt ist aus der Gruppe bestehend aus Na-Dehydrocholat und Ursodesoxycholsäure.

11. Kit nach Anspruch 10, welcher ferner umfasst:
- ein hypourikämisches Mittel, wobei das hypourikämische Mittel vorzugsweise gereinigte Triterpene aus Centella asiatica sind; und/oder
- einen Radikalfänger, wobei der Radikalfänger vorzugsweise Selen ist; und/oder
- ein sympatholytisches Mittel, wobei das sympatholytische Mittel vorzugsweise Yohimbin ist; und/oder
- ein sympathikomimetisches Mittel, wobei das sympathikomimetische Mittel ausgewählt ist aus der Gruppe bestehend aus Phendimetrazin-bitartrat und Phendimetrazin-pamoat; und/oder
- wenigstens ein Vitamin, wobei das wenigstens eine Vitamin ausgewählt ist aus der Gruppe bestehend aus Vitamin A, Vitamin B₁, Vitamin B₆, Vitamin E und Vitamin C; und/oder
- wenigstens ein Adjuvans, ausgewählt aus der Gruppe bestehend aus einem sedativen und angstlösenden Mittel, einem Appetitzügler und einem lipolytischen Mittel.

12. Kit nach Anspruch 11, worin:
- das sedative und angstlösende Mittel vorzugsweise ein Benzodiazepin, am meisten bevorzugt Dikaliumchlorazepat ist;
- der Appetitzügler vorzugsweise ausgewählt ist aus der Gruppe bestehend aus Diethylpropion-hydrochlorid, Fenfluramin-hydrochlorid, D-Fenfluramin-hydrochlorid;
- das lipolytische Mittel vorzugsweise ausgewählt ist aus der Gruppe bestehend aus Analoga von Thyroxin, wobei Trüodthyroessigsäure am meisten bevorzugt ist.

13. Verwendung einer Zusammensetzung nach einem der Ansprüche 1 bis 9 zur Herstellung eines Medikaments zum Behandeln von Nebenwirkungen von ketogenen Diäten.

## Revendications

1. Composition pharmaceutique (A) comprenant :
un agent hypocholestérolémiant, ledit agent hypocholestérolémiant étant choisi de préférence dans le groupe constitué par le benfluorex et l'acide ursodésoxycholique ;
un agent hypotriglycéridémiant, ledit agent hypotriglycéridémiant étant de préférence le benfluorex ;
un agent lipasique et protéasique, ledit agent lipasique et protéasique étant de préférence la pancréatine IX F.U. ;
un agent hypoglycémiant, ledit agent hypoglycémiant étant de préférence la metformine et
un agent hydrocholérétique, ledit agent hydrocholérétique étant choisi de préférence dans le groupe constitué par le déshydrocholate de Na et l'acide ursodésoxycholique.

2. Composition pharmaceutique selon la revendication 1, dans laquelle :
ledit benfluorex est présent en une quantité globale de 7% à 23% en masse par rapport à la quantité totale de la composition (A) ; ladite pancréatine IX F.U. est présente en une quantité de 27% à 43% en masse par rapport à la quantité totale de la composition (A) ;
ladite metformine est présente en une quantité de 36% à 41% en masse par rapport à la quantité totale de la composition (A) ;
ledit déshydrocholate de Na est présent en une quantité de 9% à 14% par rapport à la quantité totale de la composition (A).

3. Composition pharmaceutique selon la revendication 1, dans laquelle :
ledit benfluorex est présent en une quantité globale de 7% à 18% en masse par rapport à la quantité totale de la composition (A) ;
ladite pancréatine IX F.U. est présente en une quantité de 22% à 43% en masse par rapport à la quantité totale de la composition (A) ;
ladite metformine est présente en une quantité de 33% à 36% en masse par rapport à la quantité totale de la composition (A) ;
ledit acide ursodésoxycholique est présent en une quantité de 14% à 17% par rapport à la quantité totale de la composition (A).

4. Composition pharmaceutique selon l'une quelconque des revendications 1 à 3, dans laquelle la composition (A) comprend en outre :
- un agent hypouricémiant, ledit agent hypouricémiant étant de préférence des triterpènes purifiés à partir de *Centella asiatica* ; et/ou
- un agent piégeant les radicaux, ledit agent piégeant les radicaux étant de préférence le sélénium; et/ou
- un agent sympatholytique, ledit agent sympatholytique étant de préférence la yohimbine ; et/ou
- un agent sympathomimétique, ledit agent sympathomimétique étant choisi dans le groupe constitué par le bitartrate de phendimétrazinium et le pamoate de phendimétrazinium ; et/ou
- au moins une vitamine, ladite au moins une vitamine étant choisie dans le groupe constitué par la vitamine A, la vitamine B₁, la vitamine B₆, la vitamine E et la vitamine C.

5. Composition pharmaceutique selon l'une quelconque des revendications 1 à 4, la composition comprenant en outre au moins un adjuvant de régime choisi dans le groupe constitué par des agents sédatifs-anxiolytiques, des agents anorexigènes et des agents lipolytiques.

6. Composition pharmaceutique selon la revendication 4 ou 5, comprenant :
- des triterpènes purifiés à partir de *Centella asiatica* dans un rapport de 0,04:1 à 0,5:1 en masse par rapport à la masse totale de la composition (A) des revendications 1 à 3 ; et/ou
- du sélénium dans un rapport de 0,09:1 à 0,3:1 en masse par rapport à la masse totale de la composition (A) des revendications 1 à 3 ; et/ou
- de la yohimbine dans un rapport de 0,0009:1 à 0,007:1 en masse par rapport à la masse totale de la composition (A) des revendications 1 à 3 ; et/ou
- du bitartrate de phendimétrazine ou du pamoate de phendimétrazine dans un rapport de 0,004:1 à 0,13:1 en masse par rapport à la masse totale de la composition (A) des revendications 1 à 3 ; et/ou
- de la vitamine A dans un rapport de 0,5:1 à 1,8:1 en masse par rapport à la masse totale de la composition (A) des revendications 1 à 3 ; et/ou
- de la vitamine B₁ dans un rapport de 0,002:1 à 0,007:1 en masse par rapport à la masse totale de la composition (A) des revendications 1 à 3 ; et/ou
- de la vitamine B₆ dans un rapport de 0,05:1 à 0,2:1 en masse par rapport à la masse totale de la composition (A) des revendications 1 à 3 ; et/ou
- de la vitamine E dans un rapport de 0,09:1 à 1:1 en masse par rapport à la masse totale de la composition (A) des revendications 1 à 3 ; et/ou
- de la vitamine C dans un rapport de 0,09:1 à 0,3:1 en masse par rapport à la masse totale dé la composition (A) des revendications 1 à 3.

7. Composition pharmaceutique selon la revendication 5 ou 6, dans laquelle :
- ledit agent sédatif-anxiolytique est de préférence une benzodiazépine, le plus préférablement le chlorazépate dipotassique, dans un rapport de 0,0005:1 à 0,03:1 en masse par rapport à la masse totale de la composition (A) des revendications 1 à 3 ; et/ou
- ledit agent anorexigène est de préférence choisi dans le groupe constitué par le chlorhydrate de diéthylpropione, le chlorhydrate de fenfluramine, le chlorhydrate de D-fenfluramine, ledit agent anorexigène étant présent dans un rapport de 0,002:1 à 1,3:1 en masse par rapport à la masse totale de la composition (A) des revendications 1 à 3 ; et/ou
- ledit agent lipolytique est de préférence choisi dans le groupe constitué par les analogues de la thyroxine, le plus préférablement l'acide triiodothyroacétique, présent dans un rapport de 0,0002:1 à 0,003:1 en masse par rapport à la masse totale de la composition (A) des revendications 1 à 3.

8. Composition pharmaceutique selon l'une quelconque des revendications 1 à 7, comprenant en outre des excipients appropriés pharmaceutiquement acceptables.

9. Composition pharmaceutique selon l'une quelconque des revendications 1 à 8, ladite composition étant une composition solide pour administration orale comprenant des comprimés, des poudres à disperser, des granulés et des gélules en gélatine.

10. Kit de parties pour une administration simultanée, séquentielle ou séparée, comprenant :
un agent hypocholestérolémiant, ledit agent hypocholestérolémiant étant choisi de préférence dans le groupe constitué par le benfluorex et l'acide ursodésoxycholique ;
un agent hypotriglycéridémiant, ledit agent hypotriglycéridémiant étant de préférence le benfluorex ;
un agent lipasique et protéasique, ledit agent lipasique et protéasique étant de préférence la pancréatine IX F.U. ;
un agent hypoglycémiant, ledit agent hypoglycémiant étant de préférence la metformine et
un agent hydrocholérétique, ledit agent hydrocholérétique étant choisi de préférence dans le groupe constitué par le déshydrocholate de Na et l'acide ursodésoxycholique.

11. Kit selon la revendication 10, comprenant en outre :
- un agent hypouricémiant, ledit agent hypouricémiant étant de préférence des triterpènes purifiés à partir de *Centella asiatica* ; et/ou
- un agent piégeant les radicaux, ledit agent piégeant les radicaux étant de préférence le sélénium ; et/ou
- un agent sympatholytique, ledit agent sympatholytique étant de préférence la yohimbine; et/ou
- un agent sympathomimétique, ledit agent sympathomimétique étant choisi dans le groupe constitué par le bitartrate de phendimétrazinium et le pamoate de phendimétrazinium ; et/ou
- au moins une vitamine, ladite au moins une vitamine étant choisie dans le groupe constitué par la vitamine A, la vitamine B₁, la vitamine B₆, la vitamine E et la vitamine C ; et/ou
- au moins un adjuvant choisi dans le groupe constitué par un agent sédatif-anxiolytique, un agent anorexigène et un agent lipolytique.

12. Kit selon la revendication 11, dans lequel :
- ledit agent sédatif-anxiolytique est de préférence une benzodiazépine, le plus préférablement le chlorazépate dipotassique ;
- ledit agent anorexigène est de préférence choisi dans le groupe constitué par le chlorhydrate de diéthylpropione, le chlorhydrate de fenfluramine, le chlorhydrate de D-fenfluramine ;
- ledit lipolytic agent est de préférence choisi dans le groupe constitué par les analogues de la thyroxine, le plus préférablement l'acide triiodothyroacétique.

13. Utilisation d'une composition selon l'une quelconque des revendications 1 à 9 pour la fabrication d'un médicament pour le traitement des effets secondaires de régimes cétogènes.
